# EUROPEAN PATENT APPLICATION

(11) **EP 1 693 085 A1**
(43) Date of publication of application: **23.08.2006**
(21) Application number: 05388004.3
(22) Date of filing: 28.01.2005
(51) Int. Cl.: A61Q 11/00

(54) **A kit for cleaning teeth by chewing of a set of chewing gum pieces**

(71) Applicant: Gumlink A/S, 7100 Vejle (DK)
(72) Inventor: Kristiansen,Tove Nordestgaard, DK-7300, Jelling (DK); Gyldenvang,Lars, DK-8500 Grenaa (DK)
(74) Representative: Indahl, Peter Jensen

(57) **Abstract**

A kit for cleaning teeth by simultaneously or sequentially chewing a set of chewing gum pieces, the individual chewing gum piece included in the set comprising gum base and one or more of the following ingredients: at last one whitening agent, at least one fresh-breath agent, at least one anti-plaque agent or anti-gingivitis agent, and optionally at least one re-mineralization agent.

## Description

The present invention relates to a kit for cleaning teeth by simultaneously or sequentially chewing a set of chewing gum pieces.

Chewing gum suitable for temporary cleaning of teeth is well-known. Such chewing gum is e.g. disclosed in the patent documents US 5,380,530, US 5,693,334, US 6,365,130 B1 and US 2004/0115247 A1. However, the known types of chewing gum has hitherto only been capable to demonstrate a limited capacity for tooth cleaning for single day use or at the most a few days use when a toothbrush is accidentally not available, after which period a thorough cleaning with toothbrush and toothpaste has been required, or for use as a supplement to daily cleaning with a toothbrush and toothpaste.

Brushing of teeth with a toothbrush is, how-ever, rather rough on the teeth and especially on the gums and will eventually unavoidable lead to abrasive damage on the treated teeth or on the gums.

Moreover, for several decades a dogma has existed among professionals and also in general among adults and particularly among parents that the sole method of properly cleaning teeth is daily use of toothbrush and toothpaste.

Most people experience now and then that a toothbrush is unavailable and then have to resort to using other means, such as a chewing gum or eating lozenges or using gargle, in order to at least obtain a sensation of clean mouth. But they are clearly aware that this does not account to proper tooth-cleaning. People continue to brush teeth daily even when they experience brushing damages. They will sometimes change to use toothbrushes having softer brushes or use electrical toothbrushes in order to obtain more favourable brush movements over the teeth and gum surfaces, but they do not dispense with toothbrushing as such.

An object of the present invention is to provide a kit for tooth cleaning wherein some active substances are kept separate from other active substances prior to performing the tooth cleaning.

Consequently, the present invention relates to a kit for cleaning teeth by simultaneously or sequentially chewing a set of chewing gum pieces, the chewing gum piece included in the set comprising gum base and one or more of the following ingredients: at least one whitening agent, at least one fresh-breath agent, at least one anti-plaque agent or anti-gingivitis agent, and optionally at least one re-mineralization agent.

By using a kit of several chewing gum pieces where some of the active substances are included in one chewing gum piece and other of the active substances are included in one or more another chewing gum piece(s) it is possible to perform tooth cleaning with an efficiency that makes use of a toothbrush and toothpaste less required or even dispensable.

The dental care product provided by the kit according to the present invention presents surprisingly a possibility of not using a toothbrush and toothpaste for daily tooth cleaning. By using several pieces of chewing gum having tailored teeth cleaning properties instead of using a toothbrush allows the user to maintain a healthy mouth condition without the highly undesired toothbrush-effect of causing abrasive damage on the teeth and gingiva.

The kit according to the present invention provides the possibility of keeping active substances strictly apart until use, thereby avoiding break down of substances due to undesired reaction. The active substances can thus be kept potent until use.

The kit also provides the possibility to the user of first to have one or more active substances to affect the oral cavity by initially chewing a first piece of chewing gum from the kit for a first period of time and then subsequently chewing a second piece of chewing gum from the kit during a second period of time, thereby administering further active substances to the oral cavity. In this sequential manner it is possible to obtain an improved effect of the active substances.

The kit can also provides the possibility to the user of simultaneous or part-simultaneous - part-sequential chewing of a first piece of chewing gum from the kit and a second piece or second pieces of chewing gum from the kit, thereby administering active substances to the oral cavity. The first piece of chewing gum may remain in the mouth when the second piece is taken.

Furthermore, the kit according to the invention also provides the possibility of varying the doses of active ingredients in a simple manner. This can be done by use of one or more pieces of chewing gum with a specific dose of a specific active ingredient, as chewing one piece administer a specific dose, chewing two pieces administer a double dose, etc.

The kit according to the invention also provides the possibility of adapting the tooth cleaning provided by administering the kit to an actual current condition of the user. The user can chew one piece of chewing gum for ordinary tooth cleaning and one or more pieces of a chewing gum with a different formulation and having a specific dose of a desired active ingredient to promote one or more specific effects directed at treating the actual tooth condition of the user.

By using gum base in the pieces of chewing gum it is possible to provide a basis for a dental care product having excellent properties in respect of avoiding abrasive damage on teeth. As gum base alone does not have sufficient cleaning effect to secure an effective daily cleaning of teeth there is added a number of active ingredients to the gum base. It has been found that at last one whitening agent, at least one fresh-breath agent, at least one anti-plaque agent or anti-gingivitis agent, and optionally at least one re-mineralization agent are required in order to produce a cleaning effect which makes it possible to avoid the use of a toothbrush and yet maintain the teeth and gingiva in well cleaned condition. By dispensing with the use of a toothbrush the user obtains a major benefit in that abrasive damages caused by brushing teeth are avoided.

Although the present invention aims at a dental care product based on gum base and capable of cleaning teeth with an efficiency that is overall comparable to cleaning teeth with toothbrush and toothpaste, the use of a toothbrush might to some degree be more effective in certain of the desirable effects than the effects of using the present dental care product, but this is outbalanced by avoiding the undesired effect of abrasive damage on the teeth and gingiva.

Furthermore, it has also appeared that the kit for dental care according to the invention is much easier and more convenient- in use than the conventional toothbrush with toothpaste. This is in particular advantageously in case of children and disabled persons to whom handling of a toothbrush can be extremely difficult, which fact may very easily lead to the effect that the teeth cleaning become ineffective.

The availability of the present dental care product is much higher than for a toothbrush, because the gum will normally be carried with or on the user, whereas a toothbrush is kept in the bathroom. The dental care product according to the invention can be used anywhere at any time, as there is no need for access to water, like e.g. in a bathroom. Consequently, the dental care product according to the invention can be used when driving a car, during work, while watching television etc., thereby providing much more freedom to the user.

The individual chewing gum will typically be used for longer duration than the time spent on brushing teeth. When a toothbrush is used the recommended duration of brushing is 2 minutes, but many persons spend less time than that. After completed brushing of teeth it is common to rinse the mouth with water, and in doing so the active substances in the toothpaste are washed out from the mouth. However, when using the chewing gum according to the present invention, chewing is much more prolonged and the mouth is exposed to the active ingredients for longer time during the chewing. In addition the user will typically not rinse the mouth with water after completed chewing, and thus the active ingredients released during chewing will remain in the mouth, especially in the confined areas near the gingival facing towards the chins where the need for a cleaning effect is the most pronounced. It is also a benefit of the present invention that the active ingredients will automatically spread to all surfaces in the mouth, whereas a toothbrush is only really effective in the areas that are actually brushed by the action of the user.

People may try to combine tooth brushing with chewing of chewing gum of the kit according to the present invention in order to clean their teeth in a more gentle way. In this manner the toothbrush can be slowly phased out as the user gradually recognizes the benefits of cleaning teeth by using kits according to the present invention.

The kit according to the invention may comprise sets with two, three or more individual pieces of chewing gum having different formulations, at least in respect of active substances. In general, the set should comprise at least one piece of chewing gum for ordinary teeth cleaning. In addition the set may comprise at least one piece of chewing gum adapted for a more specific purpose in the tooth cleaning process. To obtain the effects desired in ordinary tooth cleaning, the chewing gum pieces in the set should include at least one whitening agent, at least one fresh-breath agent, and at least one anti-plaque agent or anti-gingivitis agent or at least one of both of these agents, viz. at least one anti-plaque agent and at least one anti-gingivitis agent. The chewing gum pieces can optionally include at least one re-mineralization agent to obtain better tooth maintenance properties.

Suitable whitening agents for use in the kit according to the invention can e.g. be selected from baking soda, Icelandic moss, bamboo, calcium pyrophosphate, calcium cabonate, sodium hexa-metaphosphate and/or sodium hexa-metaphosphate.

Suitable fresh-breath agents for use in the kit according to the invention can e.g. be selected from zinc acetate, coriander, green tea, propolis, tea tree oil, barberry bark, hexetidine, champes, sunphenol, applephenol, red tea, green tea extract, white tea and/or thyme extract.

Suitable anti-plaque agents for use in the kit according to the invention are agents that can be selected from e.g. zinc acetate, ammonium carbamate, eucalyptus, cranberry, xylitol, chlorhexidine, seaweed, osteopontin and/or baking soda.

Suitable anti-gingivitis agents for use in the kit according to the invention are agents that can be selected from chlorhexidine, myrrh, neem, sage, aloe vera, hexatidine, osteopontin, quince, and/or IG-LY 4023 (Tradename, immuglobuline-lysozyme powder obtainable from Pedersen's Laboratorium, Vejle, Denmark).

Suitable re-mineralisation agents for use according to the invention are agents that can be selected from calcium, fluoride, osteopontin, and/or phoscal (tradename for a chemical complex between casein phosphoprotein and nanoclusters of amorphous calcium phosphate).

For further active ingredients suitable for use in the kit according to the invention see examples later in the description.

In order to obtain the best possible tooth cleaning properties of the kit according to the invention it is preferred that at least one of the chewing gum pieces in the set includes at least one anti-calculus agent to provide an anti-calculus effect.

Suitable anti-calculus agents can e.g. be selected from vitamin C, citric acid, and acetic acid.

In a preferred embodiment the kit includes several sets of chewing gum pieces, each individual set being provided for a tooth cleaning procedure. The embodiment is an alternative to providing just a single set of chewing gum pieces in the kit. When a set comprises only few pieces of chewing gum, such as two or three pieces, the user may find it inconvenient if the complete kit only provides the user with a single set.

In a further development of this embodiment the kit includes several individual sets for use during a single day. By supplying the sets needed for performing several teeth brushing procedures in a single kit the user is given an incentive to use the several sets during one day. This can in particular be an advantage in relation to children or other persons who can have difficulties in keeping track of the number of sets utilized for cleaning teeth during the day. In this respect the user obtains the assistance of having information indicating the number of tooth cleanings performed during the day, information of a kind unavailable when using a toothbrush.

The kit can also include a plurality of sets of chewing gum pieces intended to be used during a predetermined period of time, such as one week, or one month. In such a kit the sets can be arranged on several individual sheets, such as one sheet with a number of e.g. three sets for use during a single day, and e.g. seven sheets included in the complete kit predetermined to last for one week.

When the kit for cleaning teeth according to the present invention is embodied for treating a specific teeth or mouth condition of the user, the individual set preferably includes at least one first chewing gum piece for basic teeth cleaning and at least one second chewing gum piece for treatment of a specific teeth condition. It is a distinct advantage that the user can be provided with a kit designed to treat his or hers special problems. If the condition is only slightly pronounced the set can include only a single second chewing gum piece, whereas it can include two or more pieces if the condition is more aggravated. In this manner the dosage of active agents to treat the condition can be obtained by varying the number of used standard chewing gum pieces containing the agent. This provides the manufacturer of the benefit of manufacturing one and the same piece of chewing gum for users requiring different dosages, and the user obtains the benefit of enhanced action of a particular size of dose, because the distribution of the complete dose on several pieces of chewing gum causes the user to chew the gum pieces over an extended period of time, thus prolonging the action of the active agents.

In a particularly advantageous embodiment said at least one second chewing gum piece is for treatment of gingivitis. Gingivitis is a condition that can come and go over time. If a person experiences a condition of gingivitis the embodiment allows the person to initiate a temporary use of a tooth cleaning kit according to said embodiment of the invention where the second chewing gum piece provides an enhanced level of active agents to counter gingivitis.

In another particularly advantageous embodiment said at least one second chewing gum piece is for treatment of plaque. If a person experiences a condition of plaque the embodiment allows the person to initiate a temporary use of a tooth cleaning kit according to said embodiment of the invention where the second chewing gum piece provides an enhanced level of active agents to treat plaque.

In the event a treatment of a specific mouth or teeth condition requires an increase of the dosage or the duration of active agents it is preferred that there are two or more second chewing gum pieces included in the set. If the concentration of the active agent must be increased the two or more pieces can be chewed together, and in case the duration of the action of the active agent is of importance, then the pieces can be chewed one after the other in order to obtain a prolonged tooth cleaning action.

According to the present invention the individual set of the kit can include several first chewing gum pieces for basic teeth cleaning, said first chewing gum pieces providing at last one whitening agent, at least one fresh-breath agent, at least one anti-plaque agent or anti-gingivitis agent, and at least one re-mineralization agent. In one embodiment the set includes only said first chewing gum pieces. By having a set with several of said chewing gum pieces the user obtains the possibility of either using the pieces sequentially and thus obtain a more prolonged duration of the action of the agents active for tooth cleaning, or using the pieces concurrently, or partly concurrent partly sequential, in order to both increase the dose level and increase the duration of the impact of the dose.

The invention will now be further explained with reference to examples.

Gum base refers in general to any commercially available gum base suitable for production of chewing gum. Such gum bases are well-known and available in the market and normally comprise natural and/or synthetic resins and optionally other ingredients. The gum base may be biodegradable.

Chewing gum is the final product, including gum base, active ingredients and optional other ingredients such as taste ingredients and colouring agents. The chewing gum is ready to use by the consumer for cleaning teeth.

Active therapeutic ingredient means any ingredient that has a direct therapeutic effect on the teeth and the oral environment including gingiva. Some active therapeutic ingredients may be active against more than one condition, e.g. function as both anti-plaque agent and anti-calculus agent and they are in the present context listed under both functions.

Anti-plaque agents include any agent that has a specific therapeutic effect of preventing or inhibiting plaque or of minimizing or removing existing plaque formations.

Anti-gingivitis agents include any agent that has a specific therapeutic effect of preventing or inhibiting gingivitis or of minimizing or removing existing gingivitis.

Anti-calculus agents include any agent that has a specific therapeutic effect of preventing or inhibiting calculus or of minimizing or removing existing calculus formations.

Re-mineralization agents include any agent that has a specific therapeutic effect in improving the degree of re-mineralization of the teeth or avoiding de-mineralization of the teeth.

Active cosmetic ingredients are ingredients that have cosmetic effect on the teeth or the oral cavity, i.e. to improve the appearance of the teeth including odour.

Whitening agents include any agent capable of bleaching or whiten teeth.

Fresh-breath agents include any agent that provides a fresh and pleasant-smelling breath.

All percentages (%) are weight percentages unless otherwise stated.

The chewing gum according to the invention can be conventional chewing gum pieces, compressed chewing gum tablets, sticks, centre-filled chewing gum with the centre filled with liquid, gel or powder. Moreover, the active ingredients, flavour and sweetener may be encapsulated to avoid undesired reactions during storage.

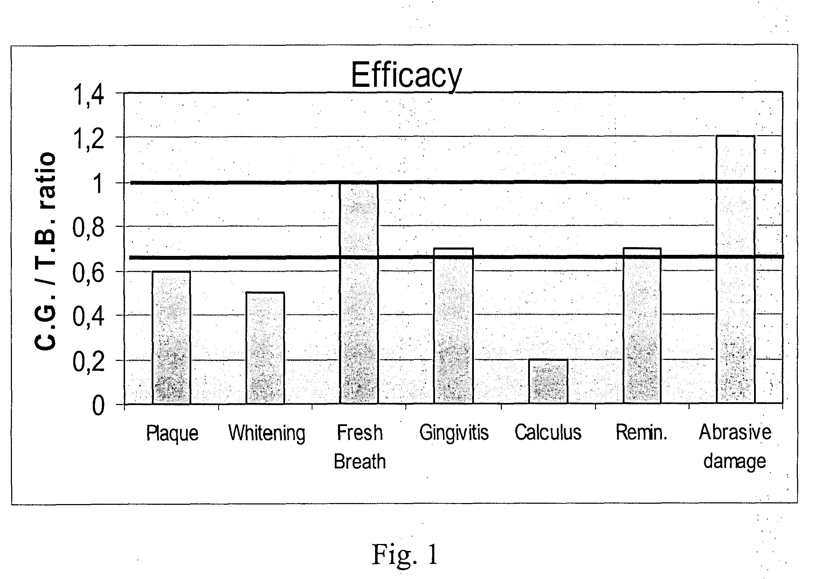

Figure 1 illustrates an estimate of the efficacy of the chewing gums tested (C.G) (chewing for 5 to 20 minutes) for dental care purposes compared with tooth brushing (T.B)(new toothbrush and correct tooth brushing for 2 minutes).

The line at 1 indicates the efficacy of a toothbrush and the columns indicates the efficacy of chewing gum in respect of plaque, whitening, fresh breath, gingivitis, calculus, re-mineralization and abrasive damage, respectively. As indicated by the line, the overall efficacy of tested chewing gum compared to tooth brushing is about 67%. The individual effects can be improved by adding more active ingredients and/or by combining ingredients so that the efficacy is raised above 70%, such as an efficacy that exceeds 100% or better efficacy than tooth brushing.

In respect of plaque (efficacy approx 60% for the tested chewing gum) the removal of plaque and/or inhibition of plaque formation can been improved, e.g, by adding zinc acetate to the chewing gum, which will enhance the effect to be close to or better than the efficacy for brushing teeth. In addition, the chewing gum according to the invention will be better than brushing teeth in real life due to the fact that the effect from chewing gum reaches places the toothbrush cannot reach. An in vivo plaque study performed by the inventors shows that e.g. zinc acetate worked in the "hard-to-reach" places with high efficiency.

Moreover, a clinical test has demonstrated that chewing gum with calcium pyrophosphate clinically whitens teeth. Presently, the effect almost matches the effect of a toothbrush with toothpaste. However, by using other agents the whitening effect will reach 100% as compared with tooth brushing.

The fresh breath effect is already better than if brushing teeth, as the chewing gum according to the invention has a much longer contact time with the volatile sulphur compounds to be eliminated. Correct tooth brushing last 2 minutes whereas chewing of chewing gum typically last for at least 5 minutes.

With respect of the anti-gingivitis effect, the chewing gum according to the invention already match this effect compared to brushing of teeth, as the contact time is longer as with brushing teeth, thereby allowing the active substances longer time to affect the infected gingiva.

As chewing gum stimulates saliva, and a wide range of active ingredients can be added to the different chewing gum pieces in the kit according to the invention that promotes re-mineralization or alternatively inhibits demineralization, the re-mineralization effect of the chewing gum exceeds the effect of brushing of teeth.

Chewing gum substantially has no abrasive effect on teeth and this parameter is naturally dramatically better than brushing teeth.

### Example

The chewing gum pieces in the following example were manufactured from a commercially available gum base (Danfree, available from Gumlink A/S, Vejle, Denmark) mixed with sweeteners, taste ingredients and active ingredients. Two different pieces of chewing gum were manufactured with ingredients in the following ratios:

| Chewing gum piece 1: | |
|---|---|
| Gum base | 60.00% |
| Sorbitol | 18.20% |
| Peppermint powder | 1.50% |
| Menthol powder | 0.30% |
| Dicalciumphosphate | 2.70% |
| Green tea | 5.00% |
| Baking soda | 0.40% |
| Calcium carbonate | 4.10% |
| Calcium pyrophosphate | 6.50% |
| Succralose | 0.25% |
| Magnesium stearate | 0.50% |
| Eucalyptus powder | 0.50% |

| Chewing gum piece 2: | |
|---|---|
| Gum base | 30.00% |
| Xylitol | 68.80% |
| Peppermint powder | 0.50% |
| Menthole powder | 0.20% |
| Magnesium stearate | 0.50% |

The gum base was granulated (GALA underwater pelletizer) to form granules with diameters in the range of approximately 0.5 - 1.5 mm and mixed with the active ingredients.

The particulate mixture of formulation 1 (1.5 g) was filled into a tablet pressing machine and compressed to form a first layer. Then 0.7 g pure gum base granules were filled into the tablet pressing machine and compressed onto the first layer to form a barriere layer. Finally 2 g of formulation 2 particulate material was filed into the tablet pressing machine and compressed.

The resulting cylindrical shaped layered chewing gum tablets had an average weight of about 4.2 g and a diameter of about 8 mm.

The chewing gum was evaluated for inhibition of plaque formation in a clinical study.

The test subjects abstained from all oral hygiene for 2 days and either chewed the gum five times per day or used no gum (Plaque scores were assigned using the Modified Quickly-Hein (MQH) index). The result demonstrated that chewing gum comprising xylitol was significantly more effective in inhibiting the formation of plaque on teeth when used as the only means of oral hygiene for two days. Additionally, it was most efficient in areas that are often missed during tooth brushing.

In conclusion, the results demonstrate that the chewing gum containing xylitol is able to reduce dental plaque formation. Moreover, the chewing gum has an ability to make dental plaque less adhesive and thus easier to remove during chewing. As a further benefit, xylitol inhibit bacterial growth and thereby inhibit tooth decay.

The chewing gum was also evaluated for its whitening effect. The chewing gum comprising calcium pyrophosphate not only results in whiter teeth by stain removal, it also helps to prevent further stain after consumption of foods and beverages.

Clinical studies on the inhibition of stain over a 14 days period showed that when chewing, chewing gum according to the invention 20 minutes each day, compared to chewing, chewing gum with 4.5% calcium carbonate, commercially available on the market, the inhibition of stain was considerably improved.

The dicalcium phosphate in the chewing gum improves the re-mineralization rate of the teeth.

Green tea provided excellent fresh breath properties in the chewing gum.

In the following further examples of ingredients are mentioned.

Anti-plaque agents include fluoride ion sources. Anti-plaque agents are any substance which by itself acts to inhibit the accumulation of bacterial deposits on the surfaces of the oral cavity. Examples include xylitol and other anti-microbial agents. The inhibition effects of the xylitol on oral microbes may have better effect when used in conjunction with an extract since the extract is also acting to disable the microbes.

Typical examples of active ingredients that are particularly desirable from considerations of anti-plaque effectiveness, safety and formulation are:
Naficillin, oxacillin, vancomycin, clindamycin, erythromycin, trimethoprim-sulphamethoxazole, rifampin, ciprofloxacin, broad spectrum penicillin, amoxicillin, gentamicin, ceftriazoxone, cefotaxime, chloramphenicol, clavunate, sulbactam, probenecid, doxycycline, spectinomycin, cefixime, penicillin G, minocycline, .beta.-lactamase inhibitors; meziocillin, piperacillin, aztreonam, norfloxacin, trimethoprim, ceftazidime, dapsone. Halogenated diphenyl ethers, e.g. 2',4,4'-trichloro-2-hydroxydiphenyl ether (Triclosan), 2,2'-dihydroxy-5,5'-dibromo-diphenyl ether. Haloqenated salicylanilides, e.g. 4',5-dibromosalicylanilide, 3,4',5-trichloro-salicylanilide, 3,4',5-tribromo-salicylanilide, 2,3,3',5-tetrachloro-salicylanilide, 3,3,3',5-tetrachloro-salicylanilide, 3,5-dibromo-3'-trifluoromethyl-salicylanilide, 5-n-octanoyl-3'-trifluoromethyl-salicY:larlilide, 3,5-dibromo-4'-trifluoromethyl-salicylariilide, 3,5-dibromo-3'-trifluoromethyl-salicylanilide (Flurophene). Benzoic esters, e.g. methyl-p-hydroxybenzoic ester, ethyl-p-hydroxybenzoic ester, propyl-p-hydroxybenzoic ester, butyl-p-hydroxybenzoic ester. Halogenated carbanilides, e.g. 3,4,4'-trichlorocarbanilide, 3-trifluoromethyl-4,4'-dichlorocarbanilide, or 3,3,4' - trichlorocarbanilide. Phenolic compounds (including phenol and its homologs, mono- and poly-alkyl and aromatic halo-phenol and their homologs), e.g. phenol, 2-methyl-phenol, 3-methyl-phenol, 4-methyl-phenol, 4-ethyl-phenol, 2,4-dimethyl-phenol, 2,5-dimethyl-phenol, 3,4-dimethyl-phenol, 2,6-dimethyl-phenol, 4-n-propyl-phenol, 4-n-butyl-phenol, 4-n-amyl-phenol, 4-tert-amyl-phenol, 4-n-hexyl-phenol, 4-n-heptyl-phenol, 2-methoxy-4-(2-propenyl)-phenol (Eugenol), 2-isopropyl-5-methyl-phenol (Thymol), mono- and poly-alkyl- and aralkyl-halophenols, methyl-p-chlorophenol, ethyl-p-chlorphenol, n-propyl-p-chlorophenol, n-butyl-p-chlorophenol, n-amyl-p-chlorophenol, sec-amyl-p-chlorophenol, n-hexyl-p-chlorophenol, cyclohexyl-p-chlorophenol, n-heptyl-p-chlorophenol, n-octyl-p-chlorophenol, o-chlorophenol, methyl-o-chlorophenol, ethyl-o-chlorophenol, n-propyl-o-chlorophenol, n-butyl-o-chlorophenol, n-amyl-o-chlorophenol, tert-amyl-o-chlorophenol, n-hexyl-o-chlorophenol, n-heptyl-o-chloropenol, p-chlorophenol, o-benzyl-p-chlorophenol, o-benzyl-m-methyl-p-chlorophenol, o-benzyl-m,m-dimethyl-p-chlorophenol, o-phenylethyl-p-chlorophenol, o-phenylethyl-m-methyl-p-chlorophenol, 3-methyl-p-chlorophenol, 3,5-dimethyl-p-chlorophenol, 6-ethyl-3-methyr-p-chlorophenol, 6-n-propyl-3-methyl-p-chlorophenol, 6-iso-propyl-3-methyl-p-chlorophenol, 2-ethyl-3,5-dimethyl-p-chlorophenol, 6-sec-butyl-3-methyl-p-chlorophenol, 2-iso-propyl-3,5-dimethyl-p-chlorophenol, 6-diethylmethyl-3-methyl-p-chlorophenol, 6-iso-propyl-2-ethyl-3-methyl-p-chlorophenol, 2-sec-amyl-3,5-dimethyl-p-chlorophenol, 2-diethylmethyl-3,5-dimethyl-p-chlorophenol, 6-secoctyl-3-methyl-p-chlorophenol, p-bromophenol, methyl-p-bromophenol, ethyl-p-bromophenol, n-propyl-p-bromophenol, n-butyl-p-bromophenol, n-amyl-p-bromophenol, sec-amyl-p-bromophenol, n-hexyl-p-bromophenol, cyclohexyl-p-bromophenol, o-bromophenol, tert-amyl-o-bromophenol, n-hexyl-o-bromophenol, n-propyl-m,m-dimethyl-o-bromophenol, 2-phenyl-phenol, 4-chloro-2-methyl-phenol, 4-chloro-3-methyl-phenol, 4-chloro-3,5-dimethyl-phenol, 2,4-dichloro-3,5-dimethyl-phenol, 3,4,5,6-tetrabromo-2-methylphenol, 5-methyl-2-pentylphenol 4-isopropyl-3-methylphenol 5-chloro-2-hydroxydiphenyl-methane. Resorcinol and its derivatives, e.g. resorcinol, methyl-resorcinol, ethyl-resorcinol, n-propyl-resorcinol, n-butyl-resorcinol, n-amyl-resorcinol, n-hexyl-resorcinol, n-heptyl-resorcinol, n-octyl-resorcinol, n-nonyl-resorcinol, phenyl-resorcinol, benzyl-resorcinol, phenylethyl-resorcinol, phenylpropyl-resorcinol, p-chlorobenzyl-resorcinol, 5-chloro-2,4-dihydroxydiphenyl-methane, 4'-chloro-2,4-dihydroxydiphenyl-methane, 5-bromo-2,4-dihydroxydiphenyl-methane, 4"-bromo-2,4-dihydroxydiphenyl-methane. Bisphenolic compounds, e.g. bisphenol A, 2,2'-methylene-bis-(4-chlorophenol), 2,2'-methylene-bis-(3,4,6-trichlorophenol) (hexachlorophene), 2,2'-methylene-bis-(4-chloro-6-bromophenol), bis-(2-hydroxy-3,5-dichlorophenyl)-sulfide, bis-(2-hydroxy-5-chlorobenzyl)-sulfide.

Illustrative of polyphosphate compounds with plaque-inhibiting properties are dialkali metal and tetraalkali metal pyrophosphate and mixtures thereof in a hydrated or unhydrated form. Illustrative of pyrophosphate salts are Na₂H₂P₂O₇, Na₄P₂O₇ and K₄P₂O₇. Other suitable polyphosphates include hydrated or unhydrated alkali metal tripolyphosphates such as Na₅P₃O₁₀ and K₅P₃O₁₀.

Plaque buffers such as urea, calcium lactate, calcium glycerophosphate and strontium polyacrylates, ammonium carbonate and vitamins such as Vitamins A, C and E are also included.

Nutraceuticals and nutritional supplements may also be added to chewing gums as active agents against plaque. Among these are herbs and botanicals that include, but are not limited to chamomile, echinacea, Eucalyptus and green tea.

Metal cations can also be used as antibacterial and anti-plaque agents. The metal cations are selected from the metals of Group 5 (V, Nb, Ta); Group 6 (Cr, Mo, W); Group 7 (Mn, Tc, Re); Group 8 (Fe, Ru, Os); Group 9 (Co, Rh, Ir); Group 10 (Ni, Pd, Pt); Group 11 (Cu, Ag, Au); Group 12 (Zn, Cd, Hg); Group 14 (Ge, Sn, Pb); Group 16 (Se, Te, PO); and mixtures thereof. Preferably the metal cation is selected from any monovalent or divalent cation selected from the group consisting of zinc, manganese, copper, iron, cobalt, silver, selenium, tin and vanadium; preferably from the group consisting of zinc, manganese, copper, iron, silver, and tin; more preferably from the group consisting of zinc, copper, silver and tin and most preferably from the group consisting of zinc and tin.

Illustrative of zinc compounds with plaque-inhibiting properties are zinc oxide, zinc silicate, zinc carbonate, zinc phosphate, zinc stannate, zinc tetrafluoroborate, zinc hexafluorosilicate, zinc citrate, zinc benzoate, zinc oxalate, zinc stearate, zinc chloride, zinc sulfate, zinc nitrate, zinc phenolsulfonate, zinc carboxymethylsuccinate, and the like. The zinc compound also can be in the form of a complex, with a complexing reagent such as polyethylenimine or ethylenediamine tetraacetic acid.

A wide variety of metal cation salts are useful in the present invention. These include so called "water-insoluble salts" which have a solubility of less than about 0.5 g per 100 ml at 25 degree C and "water soluble salts" which have a solubility of greater than or equal to about 0.5 g per 100 ml at 25 degree C. It is also possible to use mixtures of these salts. Such mixtures can have several advantages in the compositions of the present invention since they are likely to have different complexing properties with the polyphosphate anions. In addition they have different release rates in the saliva and can therefore act to provide controlled release profiles. Examples of salts that are suitable for use herein include acetate, ammonium sulphate, bromide, chloride, chromate, citrate, dithionate, fluorosilicate, tartrate, fluoride, formate, iodide, nitrate, phenol sulphate, salicyclate, sulphate, gluconate, succinate, glycerophosphate, lactate and mixtures thereof.

Anti-gingivitis agents can be anti-inflammatory agents, such as salicylic acid derivatives (e.g. aspirin), paraminophenol derivative (e.g. acetaminophen), indole and indene acetic acids (indo-methacin, sulindac and etodalac), heteroaryl acetic acids (tolmetin, diclofenac and ketorolac), aryl propionic acid derivatives (ibuprofen, naproxen, ketoprofen, fenopren, oxaprozine), anthranilic acids (mefenamic acid, meclofenamic acid), enolic acids (piroxicam, tenoxicam, phenylbutazone and oxyphentha-trazone), lactic acid bacteria (LAB), Osteopontin (ONP), IG-Lyt, hexefine, Aloe Vera, chlorhexedine, myrrh, or sage.

Anti-gingivitis agents also comprise psychotherapeutic agents, such as thorazine, serentil, mellaril, millazine, tindal, permitil, prolixin, trilafon, stelazine, suprazine, taractan, navan, clozaril, haldol, halperon, loxitane, moban, orap, risperdal, alprazolam, chlordiaepoxide, clonezepam, clorezepate, diazepam, halazepam, lorazepam, oxazepam, prazepam, buspirone, elvavil, anafranil, adapin, sinequan, tofranil, surmontil, asendin, norpramin, pertofrane, ludiomil, pamelor, vivactil, prozac, luvox, paxil, zoloft, effexor, welibutrin, serzone, desyrel, nardil, parnate, or eldepryl.

Anti-calculus agents suitable for use in the chewing gum according to the invention include phosphates, pyrophosphates, alkali-metal pyrophosphates, polyphosphates, phosphonates, polyphosphonates and mixtures of any of these. Pyrophosphates are among the best known for use in dental care products. The pyrophosphate salts useful in the present invention include the di-alkali metal pyrophosphate salts; tetra-alkali metal pyrophosphate salts and mixtures of any of these in their unhydrated as well as hydrated forms are the preferred species. Di-sodium dihydrogen pyrophosphate (Na₂H₂P₂O₇), tetra-sodium pyrophosphate (N₄P₂O₇), and tetrapotassium pyrophosphate (K₄P₂O₇) and mixtures thereof are specific examples.

Additional suitable anti-calculus agents include polyacrylates and other polycarboxylates, such as those disclosed in US Patent No. 3,429,963, US Patent No. 4,304,766, and US Patent No. 4,661,341, polyepoxysuccinates, such as those disclosed in US Patent No. 4,846,650, ethylenediaminetetraacetic acid as disclosed in British Patent No. 490,384, nitrilotriacetic acid and related compounds as disclosed in US Patent No. 3,678,154, polyphosphonates as disclosed in US Patent No. 3,737,533, US Patent No. 3,988,443, and US Patent No. 4,877,603.

The re-mineralisation agents are preferably pH adjusting agents, which may also be added to make the composition safe for oral tissues. These pH adjusting agents, or buffers, can be any material that is suitable to adjust the pH of the composition. Suitable materials include sodium bicarbonate, sodium phosphate, sodium hydroxide, ammonium hydroxide, potassium hydroxide, sodium stannate, triethanolamine, citric acid, hydrochloric acid, sodium citrate, calcium, fluoride, Phoscal, dicalcium phosphate, Osteopontin (ONP), monosodium phosphate, trisodium phosphate, sodium hydroxide, sodium carbonate, pectin, benzocaine, analgesics, sanguinarine extract, metronidazole, strontium chloride, potassium nitrate, carrageenan, cough and cold remedies, and the like.

The preferred active therapeutic ingredients listed above have all demonstrated good effect in improving teeth and oral health.

The preferred active cosmetic ingredients are listed below. The mentioned ingredients have all proven to provide excellent cosmetic effects.

The whitening agents are conveniently selected from teeth colour modifying substances that may be considered among the oral care actives useful in the chewing gum according to the invention. These substance are suitable for modifying the colour of the teeth to satisfy the consumer such as those listed in the CTFA Cosmetic Ingredient Handbook, 3.sup.rd Edition, Cosmetic and Fragrances Association Inc., Washington D.C. (1982), incorporated herein by reference. Specific examples include talc, mica, magnesium carbonate, calcium carbonate, calcium pyrophosphate, Baking soda, Icelandic moss, bamboo, sodium hexa-metaphosphate, magnesium silicate, aluminium magnesium carbonate, silica, titanium dioxide, zinc oxide, red iron oxide, brown iron oxide, yellow iron oxide, black iron oxide, ferric ammonium ferrocyanide, manganese violet, ultramarine, nylon powder, polyethylene powder, methacrylate powder, polystyrene powder, silk powder, crystalline cellulose, starch, titanated mica, iron oxide titanated mica, bismuth oxychloride, and mixtures thereof. Typical levels are from about 0.05% to about 20%, preferably from about 0.1% to about 15% and most preferably from about 0.25% to about 10%, by weight, of the composition.

Whitening agents for use herein may also comprise materials that remove or bleach intrinsic or extrinsic stains on or in tooth surfaces. Such substances are selected from the group consisting of the peroxides, metal chlorites, perborates, percarbonates, peroxyacids, persulphates, and combinations thereof. Suitable peroxide compounds include hydrogen peroxide, urea peroxide, calcium peroxide, carbamide peroxide and mixtures thereof. Suitable metal chlorites include calcium chlorite, barium chlorite, magnesium chlorite, lithium chlorite, sodium chlorite and potassium chlorite. Additional bleaching substances may be hypochlorite, and chlorine dioxide. A preferred percarbonate is sodium percarbonate. Preferred persulphates are oxones. The content of these substances is dependent on the available oxygen or chlorine. The content of these ingredients in the chewing gum according to the invention is generally in the range from about 0.1% to about 35%, preferably from about 1% to about 25% and most preferably from about 5% to about 10%, by weight of the chewing gum.

The fresh-breath agents are preferably selected from agent for oral malodour control, which include a wide variety of materials. Suitable in the chewing gum according to the invention are anti-microbial agents. Such agents may include 5-chloro-2-(2,4-dichlorophenoxy)-phenol, commonly referred to as triclosan, and described in the Merck Index, 11^{th} Edition, (1989), pp1529 (entry No. 9573) in US Patent No. 3,506,720, and in European Patent publication No. 0 251 591, phthalic acid and its salts including, but not limited to those disclosed in US Patent No. 4,994,262, preferably magnesium mono-potassium phthalate, chlorohexidine (Merck Index, No. 2090), alexidine (Merck Index, No. 222), hexetidine (Merck Index, No. 4624), sanguinarine (Merck Index, No. 8320), benzalkonium chloride (Merck Index, No. 1066), salicylanilide (Merck Index, No. 8299), domiphen bromide (Merck Index, No. 3411), cetylpyridinium chloride (CPC) (Merck Index, No. 2024), tetradecylpyridinium chloride (TPC), N-tetradecyl-4-ethylpyridinium chloride (TDEPC), octenifine, delmopinol, octapinol, and other piperidine derivatives, nicin preparations, zinc/stannous ion agents, antibiotics, such as augmentin, amoxicilline, tetracycline, doxycycline, hexadine, minocycline, and metronidazole, and analogues and salts of the above, methyl salicyclate, and mixtures of any of the above.

Illustrative zinc compounds with fresh breath properties for use as fresh-breath agents are zinc oxide, zinc silicate, zinc carbonate, zinc phosphate, zinc stannate, zinc tetrafluoroborate, zinc hexafluorosilicate, zinc citrate, zinc benzoate, zinc oxalate, zinc stearate, zinc chloride, zinc sulfate, zinc nitrate, zinc phenolsulfonate, zinc carboxymethylsuccinate, and the like. The zinc compounds may also be present as a complex, with a complexing agent such as polyethylenimine or ethylenediamine tetraacetic acid.

A further group of natural extracts which are useful for their oral malodour control benefits include extracts obtained from the tea (green tea, red tea, white tea and black tea), honey suckle, coriander, thyme, propolis, tea tree oil, barberry bark, champex® , sunphenon, applephenon, gold thread, magnolia plants or mixtures thereof. Extracts suitable for use in the present invention can be obtained from any part of the plant including the leaf, stem, bark, pulp, seed, flesh, juice, root and mixtures thereof. It is preferred that chewing gum according to the present invention comprise from about 0.01% to about 5%.

The following essential oils are also known to have anti-microbial activity and are therefore optionally used in chewing gum according to the present invention. These oils include thymol, geraniol, carvacrol, hinokitiol, eucalyptol, catechol (particularly 4-allyl catechol), and mixtures thereof.

Another class of oral malodour control agents include absorbents. These are used to absorb, adsorb, bind or otherwise complex the volatile oral malodour materials. Examples of such agents include talc, mushroom extract, zeolite, cyclodextrin, silica shell and mixtures thereof. Such materials are preferably used in a range from about 0.5% to about 10%, preferably from about 1% to about 5%, by weight of the chewing gum.

Typically, the chewing gum comprises a water-soluble bulk portion, a water-insoluble chewable gum base portion and typically water-insoluble flavouring agents. The water-soluble portion dissipates with a portion of the flavouring agent over a period of time during chewing. The gum base portion is retained in the mouth throughout the chew.

The insoluble gum base typically comprises elastomers, resins, fats and oils, waxes, softeners ) and inorganic fillers. Elastomers may include polyisobutylene, isobutylene-isoprene copolymer and styrene butadiene rubber, as well as natural latexes such as chicle. Resins may include polyvinylacetate and terpene resins. Fats and oils may also be included in the gum base, including tallow, hydrogenated and partially hydrogenated vegetable oils, and cocoa butter. Commonly employed waxes include paraffin, microcrystalline and natural waxes such as beeswax and carnauba.

According to an embodiment of the present invention, the insoluble gum base constitutes between about 5 to about 95 percent by weight of the gum. More preferably the insoluble gum base comprises between 10 and 50 percent by weight of the gum and most preferably about 20 to about 35 percent by weight of the gum.

Particularly interesting elastomeric or resinous polymer compounds which advantageously can be used in a process according to the invention include polymers which, in contrast to currently used elastomers and resins, can be degraded physically, chemically or enzymatically in the environment after use of the chewing gum, thereby giving rise to less environmental pollution than chewing gums based on nondegradable polymers, as the used degradable chewing gum remnants will eventually disintegrate and/or can be removed more readily by physical or chemical means from the site where it has been dumped.

Preferably if degradable, the chewing gum comprises at least two different biodegradable polymers wherein at least one of said biodegradable polymers comprises a polyester polymer.

One of said at least two different biodegradable polymers may comprise a polyester produced through reaction of at least one alcohol or derivative thereof and at least one acid or derivative thereof. Another of said at least two different biodegradable polymers may comprise a polyester obtained by polymerization of at least one cyclic ester.

The gum base typically also includes a filler component. The filler component may be magnesium and calcium carbonate, sodium sulphate, ground limestone, silicate compounds such as magnesium and aluminium silicate, kaolin and clay, aluminium oxide, silicium oxide, talc, titanium oxide, mono-, di- and tricalcium phosphates, cellulose polymers, such as wood, and combinations thereof. The filler may constitute between about 5 and about 60 percent by weight of the gum base. Preferably, the filler comprises about 5 to about 50 percent by weight of the gum base. Gum bases typically also contain softeners, including glycerol monostearate and glycerol triacetate. Furthermore, gum bases may also contain optional ingredients such as antioxidants, colours, and emulsifiers, such as lecithin, sweeteners and flavours. The present invention contemplates employing any commercially acceptable gum base.

The water-soluble portion of the chewing gum may further comprise softeners, sweeteners, flavouring agents and combinations thereof. Softeners are added to the chewing gum in order to optimize the chew ability and mouth feel of the gum. Softeners, also known in the art as plasticizers or plasticizing agents, generally constitute between about 0.1 to about 15.0 percent by weight of the chewing gum. Softeners contemplated by the present invention include glycerine, lecithin, and combinations thereof. Further, aqueous sweetener solutions such as those containing sorbitol, hydrogenated starch hydrolysates, corn syrup and combinations thereof may be used as softeners and binding agents in gum.

In a preferred embodiment of the chewing gum according to the invention the chewing gum further comprises one or more taste ingredients selected from sweeteners, high-potent sweeteners and flavours. The sweeteners may e.g. be sweeteners like sucrose, dextrose, dextrins, maltose, trehalose, D-tagatose, dried invert sugar, ribose, fructose, levulose, galactose, glucose, maltodextrin, polydextrose, isomalt, sorbitol, sorbitol syrup, mannitol, xylitol, hexa-resorcinol, maltitol, isomaltol, erythriol, lactitol, xylose, tagatose and hydrogenated starch hydrolysates (maltitol syrup). The high potent sweeteners includes the dipeptides aspartame, neotame and alitame; N-sulfonylamides such as saccharin including the salts thereof and acesulfam including the salts thereof; sulfamates such as cyclamate including the salts thereof; chlorinated sugar derivatives such as ) sucralose; Terpenoid glycosides such as Rebaudioside-A, Stevioside and Glyhyrrhizin; proteins such as thaumatin and monellin and Di-hydrochalcones.

A variety of one or more flavouring agents may be used. Flavouring agents suitable for use in the present invention include natural, natural-identical, and/or artificial flavouring substance, or mixtures thereof, in their solid and/or in their liquid state. The person skilled in the art will recognize that natural and artificial flavouring agents may be com) bined in any sensorially acceptable blends. Some examples of suitable tastes are peppermint, lemon, and orange.

When taste ingredients like sweeteners and flavours are used, these are normally admixed to the gum base or the active ingredients. Taste ingredients in the chewing gum stimulates the user to chew for a prolonged period of time, which again have the advantages that the active ingredients has a longer period to be released and affect teeth and gingival surfaces.

Consequently, the polishing material can be any material that does not abrade dental enamel and dentine. Typical materials include silica gels and precipitates, aluminas, phosphates, and mixtures thereof. Specific examples include dicalcium orthophosphate dihydrate, calcium pyrophosphate, Bamboo, tricalcium phosphate, hydrated alumina, beta calcium pyrophosphate, calcium carbonate, sodium polymeta-phosphate, sodium hexametaphosphate, Calgen, Giltex, Quadrafos, Hagan phosphate, micromet, calcium phosphate dibasic, calcium monohydrogen phosphate, dicalcium orthophosphate secondary calcium phosphate, carbonic acid calcium salt, cacti, calcichew, calcidia, citrical, aragonite, calcite, valerite, aluminum oxide, alumina, silicon dioxide, silica, silicic anhydride, and resinous abrasive materials such as particulate condensation products of urea and formaldehyde and others such as disclosed in US Patent No. 3,070,510. Mixtures of polishing agents can also be used.

The silica polishing materials generally have an average particle size ranging between about 0.1 to about 30 microns; and preferably from about 5 to about 15 microns. The polishing agent can be precipitated silica or silica gels, such as the silica xerogels described in US Patent No. 3,538,230 or in US Patent No. 3,862,307. Preferred are the silica xeropgels marketed under the name "Syloid" by the W. R. Grace and Company, Davison Chemical Division. Also preferred are the precipitated silica materials such as those marketed by the J. M. Huber Corporation under the trade name "Zeodent", particularly the silica carrying the designation "Zeodent 119". The types of silica dental polishing agents useful in the chewing gum of the present invention are described in more details in US Patent No. 4,340,583. The polishing agents in the chewing gum according to the invention is generally present in the range from about 6% to about 70%, preferably from about 10% to about 50%, by weight of the chewing gum.

The ingredients for each type of chewing gum piece in a set are mixed together, such as by initially melting the gum base and feeding it to a running mixer to which ingredients can be fed and mixed. The base may also be melted in the mixer itself. Colour or emulsifiers may also be added at this time. A softening agent such as glycerine may also be added at this time, along with syrup and a portion of the bulking agent. Further portions of the bulking agent may then be added to the mixer. A flavouring agent is typically added with the final portion of the bulking agent. Variations of the described procedure can of course be used. The chewing gum can also be manufactured from a compressed blend or mix of granules of gum base blended with powder or granules of other ingredients. Thus, particulate gum base can be mixed with particulate active agents and optionally other ingredients. The mixture is filled into a press that presses the mixture to form compressed chewing gum tablets. Use of granules is particular advantageously when one ore more of the active ingredients are sensitive towards elevated temperatures as the mixing and pressing can be done at low temperature, e.g. normal room temperature.

The set of chewing gum pieces can e.g. be packed in blister packing, and several sets can be blister packed on an insert or a sheet, e.g. with each set occupying a row on said sheet.

The different chewing gum pieces in a set can have colouring enabling the user to differentiate between the individual pieces of gum in a set. When the set includes a first chewing gum piece for basic teeth cleaning and a second chewing gum piece for treatment of a specific teeth condition, said first chewing gum piece can have a colour distinctly different from the colour of said second chewing gum piece. The kit can in this case be provided as a container with loose chewing gum pieces, and the user then picks a set of chewing gum pieces by taking e.g. one blue piece of gum and one yellow piece of gum.

Differentiation between the pieces of gum in a set can also be provided in other manners, such as by manufacturing the gum pieces in different shapes, or with markers. As an example of this a first chewing gum piece can be conventionally manufactured chewing gum, which has e.g. been scored out of a sheet of chewing gum, and a second chewing gum piece can be a chewing gum tablet of compressed particulate materials. As another example the first and second chewing gum pieces can both be chewing gum tablets, but having different diameters or different heights.

It can be a desire that the chewing gum pieces in a set are taken in a specific sequence. One chewing gum piece can e.g. include an active agent which by acting on the teeth for the some time enhance the effects of a second active agent provided in another piece of chewing gum. When such a sequential chewing of different pieces in a certain order is desired, the different chewing gum pieces in a set is preferably given a shape or colour which to the user indicates the correct sequence. As one example of this, a first chewing gum piece can be a chewing gum tablet having only a single layer (or a non-layered tablet), a second chewing gum piece can be a chewing gum tablet having two layers, and if needed a third chewing gum piece can be a chewing gum tablet having three layers etc.

Instead of layers, a user can be provided with similar information on the correct sequence by first pieces having only one colour, second pieces having two colours, and if needed a third piece having three colours etc.

A user can be alternatively be provided with similar information on the correct sequence by first pieces having only one marker, second pieces having two markers, and if needed a third piece having three markers etc. The markers can be embossed or stamped onto or into the surface of the chewing gum pieces so that the markers on the chewing gum pieces are presented to the user as markers projecting from the surface of the piece or markers recessed into the surface of the piece. The markers can have any shape or pattern considered expedient to inform the user of the suitable sequence of chewing the pieces in the set, such as actual numbers, such as 1 on the first piece, 2 on the second piece, and if required 3 on the third piece etc. The first piece can also be provided with a marker in form of one dot or slash or line, the second piece with a marker in form of two dots or two slashes or two lines etc.

A user can alternatively be provided with similar information on the correct sequence by first pieces having only one colour, second pieces having two colours, and if needed a third piece having three colours.

The pieces in a set can also be distinguished by being provided with printing onto the outer surface of the chewing gum pieces. The printing can be a numbering as just described in connection with markers, or it can be letters or text indicating to the user how the individual chewing gum piece is intended to be used in order to obtain the desired tooth cleaning effect. To give some examples, the set of chewing gum pieces can in one embodiment be composed so that the user has to take one piece of chewing gum for obtaining a normal tooth cleaning and pieces of chewing gum intended for this can be marked with the letter N (or a letter indicating "normal" in another language than English - and correspondingly for other letters) or the letter D for daily, or D1, D2, D3 if the user needs help for performing tooth cleaning three times daily. And in case the user needs tooth cleaning directed at special effects or a current condition related to the users teeth or gingiva the chewing gum pieces made for such a specific task can carry a special marking or special printing, such a M if it is for use once every Monday, G if it contains high doses of anti-gingivitis agents, P if it contains high doses of anti-plaque agents, etc.

## Claims

1. A kit for cleaning teeth by simultaneously or sequentially chewing a set of chewing gum pieces, the chewing gum pieces included in the set comprising gum base and one or more of the following ingredients: at last one whitening agent, at least one fresh-breath agent, at least one anti-plaque agent or anti-gingivitis agent, and optionally at least one re-mineralization agent.

2. A kit according to claim 1, wherein at least one of the chewing gum pieces in the set includes at least one anti-calculus agent.

3. A kit according to claim 1 or 2, wherein several sets of chewing gum pieces are included in the kit, each individual set being provided for a tooth cleaning procedure.

4. A kit according to claim 3, wherein the kit includes several individual sets for use during a single day.

5. A kit according to claim 3 or 4, wherein the kit includes a plurality of sets of chewing gum pieces intended to be used during a predetermined period of time, such as one week, or one month.

6. A kit according to any one of claims 1 to 5, wherein the individual set includes at least one first chewing gum piece for basic teeth cleaning and at least one second chewing gum piece for treatment of a specific teeth condition.

7. A kit according to claim 6, wherein said at least one second chewing gum piece is for treatment of gingivitis.

8. A kit according to claim 6, wherein said at least one second chewing gum piece is for treatment of plaque.

9. A kit according to any one of claims 6 to 8, wherein there are two or more second chewing gum pieces included in the set.

10. A kit according to any one of claims 1 to 9, wherein the individual set includes several first chewing gum pieces for basic teeth cleaning, said first chewing gum pieces providing at last one whitening agent, at least one fresh-breath agent, at least one anti-plaque agent or anti-gingivitis agent, and at least one re-mineralization agent.
